# EUROPEAN PATENT SPECIFICATION

(11) **EP 2 683 308 B1**
(45) Date of publication and mention of the grant of the patent: **10.05.2017**
(21) Application number: 12712462.6
(22) Date of filing: 09.03.2012
(51) Int. Cl.: A61B 17/17

(54) **MULTIPLE PORTAL GUIDE**
FÜHRUNG MIT MEHREN ÖFFNUNGEN
GUIDE À PORTES MULTIPLES

(30) Priority: 09.03.2011 US 201161450666 P; 09.03.2011 US 201161450669 P; 28.11.2011 US 201161563833 P
(43) Date of publication of application: 15.01.2014
(73) Proprietor: Smith & Nephew, Inc., Memphis, TN 38116 (US)
(72) Inventor: BHARAM, Srino, New York, New York 10011 (US); TORRIE, Paul Alexander, Marblehead, Massachusetts 01945 (US)
(74) Representative: Smith & Nephew
(86) International application number: PCT/US2012/028537
(87) International publication number: WO 2012/122497

(56) References cited:
- WO-A2-01/82838
- WO-A2-2006/074321
- FR-A1- 2 901 465
- US-A- 5 681 320
- US-A1- 2010 241 106

## Description

This document relates to a device for creating portals during surgery.

### BACKGROUND

During arthroscopic surgery, regions within the body, such as the hip, knee, shoulder and other joint areas, are approached via the use of an endoscope. In some joint areas, such as the hip joint, controlling the trajectory of instruments placed into the joint area for placement of portals, or tissue passages, can be difficult.

US2040241106 discloses a guide assembly which includes a guide having a body having a first set of marks and a joint including at least one through hole, the joint configured for sliding along the length of the body; and a first surgical device having a second set of marks, wherein the guide is coupled to the first surgical device and a longitudinal axis of the through hole is co-radial with a mark of the second set of marks when the joint is located at a mark of the first set of marks.

### SUMMARY

According to one aspect, a guide assembly includes a guide having a first attachment portion and a second attachment portions, an aimer arm coupled to the first attachment portion of the guide and having a distal portion configured to engage a bone surface, a surgical instrument coupled to the second attachment portion of the guide and defining a longitudinal axis that is substantially co-radial with the distal portion of the aimer arm, and one or more depth limiting elements configured to limit movement of a distal end of the surgical instrument beyond a preset location along the longitudinal axis. The distal portion of the aimer arm has an aiming feature configured to contact a bone surface. A portion of the aimer arm extends beyond the longitudinal axis of the surgical instrument. In use, a clamping force is generated between the distal portion of the aimer arm and the distal end of the surgical instrument.

Implementations of this aspect may include one or more of the following features. For example, the aiming feature may include one or more of a spike, a laser mark, or an aperture. The one or more depth limiting elements may include an area about the aiming feature. The area may be configured to receive a distal end of the surgical instrument. The one or more depth limiting elements may include a depth stop frame coupled to the guide. The depth stop frame may be configured to prevent a wide portion of the surgical instrument from advancing past the depth stop frame. The surgical instrument may further include an outer sleeve. The outer sleeve may be coupled to the guide and configured to contact a second bone surface. The second bone surface may be generally opposite the bone surface that is contact by the aiming feature of the aimer arm. The distal portion of the aimer arm may further include an aimer extension. The aimer extension may extend from the distal portion of the aimer and may be offset from the aiming feature such that a distal tip of the aimer extension lies along the longitudinal axis of the surgical instrument. The distal portion of the aimer arm may be contoured to substantially conform to a curved bone surface.

The guide assembly includes a guide having a first attachment portion and a second attachment portion, an aimer arm coupled to the first attachment portion of the guide and having a distal portion, an image capturing element coupled to the aimer arm and having a viewing direction toward the lower surface of the target portion, a surgical instrument coupled to the second attachment portion of the guide and configured to drill through bone, and one or more depth limiting elements configured to limit movement of a distal end of the surgical instrument beyond a preset location along the longitudinal axis. The distal portion of the aimer arm includes a target portion having an upper surface and a lower surface, and an aiming feature positioned on the target portion and configured to contact a bone surface. A field of view of the image capturing element includes a portion of the bone surface and a portion of the aiming feature. The surgical instrument defines a longitudinal axis that is substantially co-radial with the aiming feature.

Implementations of this aspect may include one or more of the following features. For example, the aiming feature may include one or more of a spike, a laser mark, or an aperture. The one or more depth limiting elements may include an area about the aiming feature. The area may be configured to receive a distal end of the surgical instrument. The one or more depth limiting elements may include a depth stop frame coupled to the guide. The depth stop frame may be configured to prevent a wide portion of the surgical instrument from advancing past the depth stop frame. The surgical instrument may further include an outer sleeve. The outer sleeve may be coupled to the guide and configured to contact a second bone surface. The second bone surface may be generally opposite the bone surface that is contact by the aiming feature of the aimer arm. The target portion defines a viewing window through which the image capturing element obtains a view of the portion of the bone surface and the portion of the aiming feature.

Also described herein is a method of drilling through bone includes inserting an aimer arm through a first tissue portal, engaging a bone surface with a distal portion of the aimer arm, inserting a surgical instrument through a second tissue portal and aligning the surgical instrument along the desired drilling axis, and after adjusting the guide and the surgical instrument such that the longitudinal axis is aligned with the desired drilling axis, drilling through the bone with the surgical instrument to a desired depth along the drilling axis. The aimer arm is coupled to a first attachment portion of a guide. An aiming feature of the distal portion is positioned proximate the bone surface along a desired drilling axis. The surgical instrument is coupled to a second attachment portion of the guide and defines a longitudinal axis that is substantially co-radial with the aiming feature of the distal portion of the aimer arm. A portion of the aimer arm extends beyond the longitudinal axis of the surgical instrument. One or more depth limiting elements are configured to limit movement of a distal end of the surgical instrument beyond a preset location along the longitudinal axis.

Implementations of this aspect may include one or more of the following features. For example, the surgical instrument may drill through a subchondral bone of an acetabular cup from outside a joint to an area within the joint. The distal portion of the aimer arm may be positioned under a delaminated cartilage. The one or more depth limiting elements may prevent the distal end of the surgical instrument from penetrating the delaminated cartilage. Positioning the aiming feature to be proximate the bone surface may include viewing a relative position of the aiming feature relative to the bone surface through an image capturing element coupled to the aimer arm. A field of view of the image capturing element may include a portion of the bone surface and a portion of the aiming feature. An outer sleeve may be coupled to the guide and configured to contact a second bone surface. The second bone surface may be generally opposite the bone surface that is contacted by the aiming feature of the aimer arm. A clamping force may be generated between the aiming feature and a distal tip of the outer sleeve. The distal portion of the aimer arm may engage a surface of a femoral head such that an aiming feature of the distal portion is positioned over an avascular necrosis site. The one or more depth limiting elements may prevent the distal end of the surgical instrument from penetrating a cartilage layer of the femoral head.

The details of one or more implementations are set forth in the accompanying drawings and the description below. Other features, aspects, and advantages will become apparent from the description, the drawings, and the claims.

### BRIEF DESCRIPTION OF THE DRAWINGS

FIG. 1 is a front view of a guide assembly.
FIGS. 2A and 2B illustrate positioning and use of the guide assembly within an acetabular region.
FIGS. 3A and 3B are partial perspective views of the guide assembly of FIG. 1.
FIG. 4 illustrates an example of a field of view of an image capturing element of the guide assembly of FIG. 1.
FIGS. 5A and 5B are front and partial front views of an alternative implementation of the guide assembly.
FIG. 6 is a partial front view of another alternative implementation of the guide assembly.
FIGS. 7A and 7B are partial perspective views of implementations of the guide assembly of FIG. 6.
FIGS. 8A and 8B are front and top views of another alternative implementation of the guide assembly
FIG. 9 illustrates positioning and use of the guide assembly of FIGS. 8A and 8B within a femoral head region.
FIG. 10 is a front view of another alternative implementation of the guide assembly.
FIGS. 11A and 11B are partial perspective views of another implementation of the guide assembly.
FIG. 12 is a front view of another implementation of the guide assembly.

### DETAILED DESCRIPTION

This document describes examples of a guide assembly that can be used during an arthroscopic procedure to create portals in subchondral bone, for example, to stimulate the production of fibrocartilage between an acetabular cup and a femoral head. In another example, the guide assembly can be used to treat avascular necrosis of a femoral head.

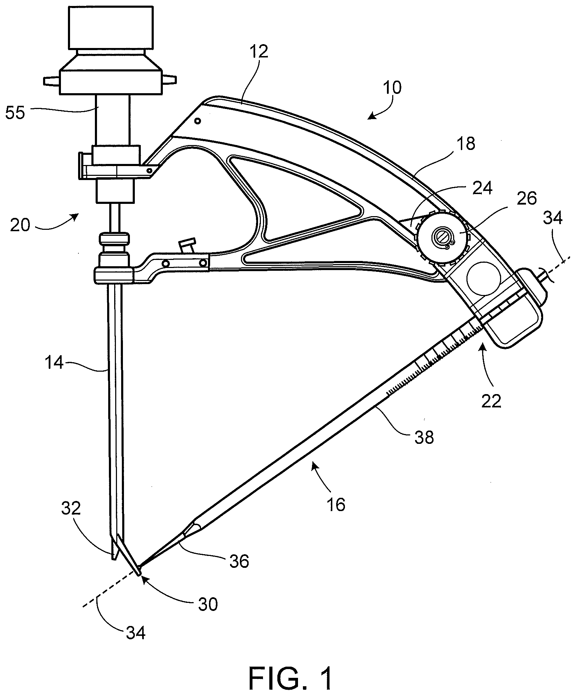

Referring to FIG. 1, a guide assembly 10 includes a guide 12, an aimer arm 14, and a surgical instrument 16. The guide assembly 10 permits a trajectory of a portal created by the surgical instrument 16 to be controlled by appropriately placing the aimer arm 14 near a surgery site, generally a joint area such as the hip joint. The guide 12, which is further described in U.S. Patent Application Serial No. 12/032,168, filed February 15, 2008, includes a body 18 having a first attachment portion 20 and a second attachment portion 22. The first and second attachment portions 20, 22 can include one or more through holes for coupling one or more various surgical devices such as an endoscope, a cannula, a drill, and the like. The body 18 of the guide 12 can further include a joint 24 that slides along the body 18 to vary a distance between the first and second attachment portions 20, 22. The joint 24 can be engaged and held in position along the body 18 using, for example, a locking mechanism 26.

The aimer arm 14 can couple to the first attachment portion 20 of the guide assembly 10 and has a distal portion 30 that is configured or adapted to contact a portion of a bone surface, as described further below. The aimer arm 14 is generally a cylindrical structure having a one-piece construction and can be made from any biocompatible material including polymers, plastics, metals, ceramics, or combinations thereof and can further be cannulated such that other surgical devices, such as an image capturing element 32 of an endoscope 55 can be disposed axially along its length.

The surgical instrument 16 can couple to the second attachment portion 22 of the guide assembly 10 and defines a longitudinal axis 34. The surgical instrument 16 is arranged relative to the aimer arm 14 such that the longitudinal axis 34 of the surgical instrument 16 intersects a portion of the distal portion 30 of the aimer arm 14. In other words, the longitudinal axis 34 defined by the surgical instrument 16 is co-radial with a portion of the distal portion 30 of the aimer arm 14. The co-radial relationship between a portion of the distal portion 30 and the surgical instrument 16 is maintained throughout the range of motion of the joint 24 along the body 18 of the guide 12. The surgical instrument 16 can include any device configured or adapted for removal of tissue or bone, for example, one or both of a drill 36 and a cannulated bullet or outer sleeve 38. In the example shown in FIG. 1, the bullet 38 movably attaches to the second attachment portion 22, and the drill 36 can be slidably disposed within the bullet 38. In use, and as described further below, a user can use the drill 36 to drill through bone along the longitudinal axis 34, and the drill 36 is limited from travelling distally beyond the distal portion 30 of the aimer arm 14.

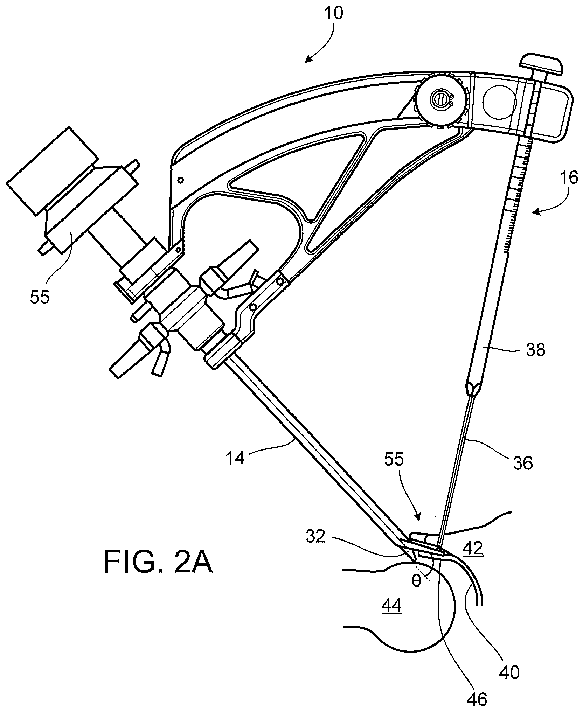

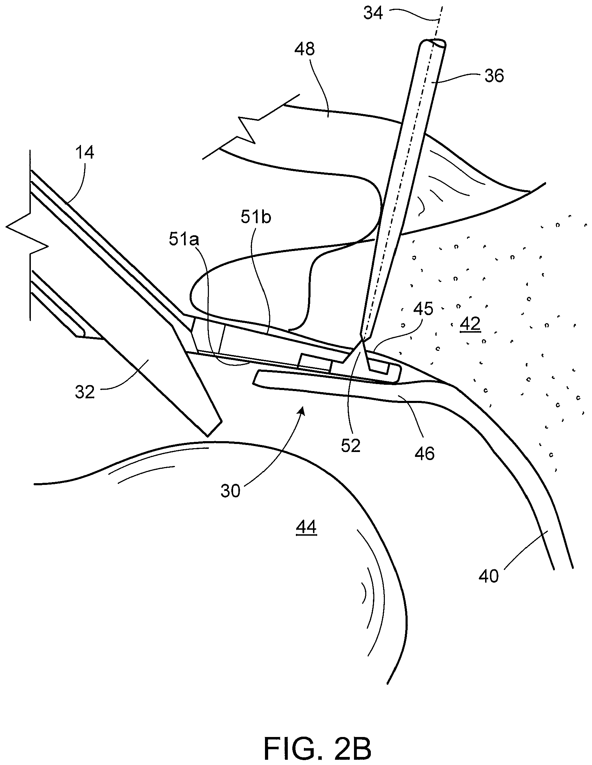

In one implementation, the guide assembly 10 can be used to drill through subchondral bone during an acetabular drilling procedure. As seen in FIGS. 2A and 2B, cartilage 40 between an acetabular cup 42 and a femoral head 44 can delaminate from the acetabulum 42. Drilling of the subchondral bone tissue lying underneath a delaminated portion 46 of the cartilage 40 can create multiple bone passages for blood and other growth factors to travel to a surface of the acetabulum 42. Such drilling can promote, for example, the growth of fibrocartilage at the joint surface. Alternatively, or additionally, drilling may also assist in the re-adhesion of the delaminated cartilage 46 to the subchondral bone of the acetabulum 42.

Referring to FIGS. 2A and 2B, in use, a user, such as a surgeon, can position the guide assembly 10 relative to a surgical site 45 of the acetabular drilling procedure such that the distal portion 30 of the aimer arm 14 slides in between a portion of the delaminated cartilage 46 and the surface of the acetabulum 42. The surgical instrument 16, such as the drill 36 and the movable bullet 38, can then be used to drill through the subchondral bone of the acetabular cup 42 from outside the joint to a point within the joint. In some cases, the surgical instrument 16 can drill through other tissues, such as a hip capsule 48, before passing through the acetabular cup 42. By contrast, in a typical microfracture procedure, bone passages are initiated from underneath the subchondral bone surface within the joint. Additionally, microfracture procedures are generally performed using non-rotating awls or picks.

To help the surgeon control the placement of the drill 36 within the acetabulum 42 relative to the surgical site 45, the distal portion 30 of the aimer arm 14 includes a target 50. The target 50 is a generally flat, paddle-like structure having a lower surface 51a, an upper surface 51b, and an aiming feature 52. The longitudinal axis 34 is substantially co-radial with the aiming feature 52, which can be a cone-like structure having a spike that is configured to contact and generally engage a bone surface. Alternatively, the aiming feature 52 can be a laser mark, or as discussed further below, an aperture. Additionally, the image capturing element 32, which can be for example, a lens or a camera end of an endoscope 55, is coupled to the aimer arm 14 and is positioned and oriented such that a viewing direction of the image capturing element 32 points toward the lower surface 51a of the target 50. As discussed further below, a resulting field of view of the image capturing element 32 includes the bone surface of the surgical site 45 and the aiming feature 52. Because the longitudinal axis 34 of the drill 36 is substantially co-radial with the aiming feature 52, the drill 36 will create the bone passage and exit the bone surface at a point where the aiming feature 52 makes contact with the bone surface.

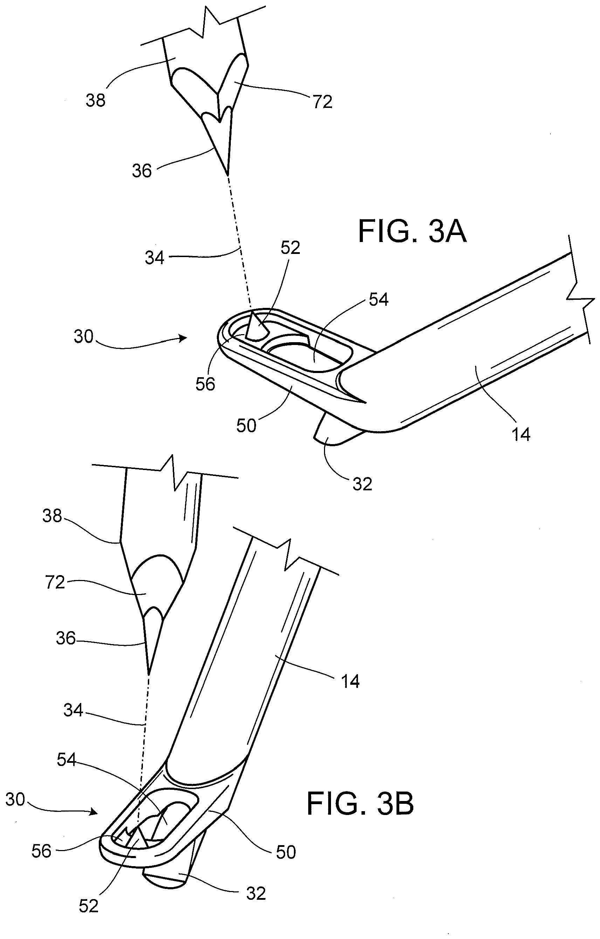

Referring also to FIGS. 3A and 3B, and as discussed above, the longitudinal axis 34 of the drill 36 is substantially co-radial with the aiming feature 52. As such, the surgeon can adjust the trajectory or drilling axis, and thus the exit point, of the drill 36 and the resulting bone passage by viewing an image of the surgical site 45 as captured by the image capturing element 32 and appropriately positioning the aiming feature 52 relative to the desired surgical site 45. To ensure that the field of view of the image capturing element 32 includes an unobstructed view of both the surgical site 45 and the aiming feature 52, the target 50 is shaped such that it does not block a line of sight from the image capturing element 32 to the aiming feature 52. The target 50 includes a viewing window 54, which can be an opening or an optically transparent region in the target 50. Additionally, a distance between the lower surface 51a and the upper surface 51b of the target 50 (FIG. 2B) can be varied to ensure that the delaminated cartilage portion 46 is sufficiently deflected out of the field of view of the image capturing element 32 and thus does not obstruct the view of the surgical site 45 and the aiming feature 52. Additionally, or alternatively, the target 50 may be made from an optically transparent material.

The target 50 is angularly offset from and forms an angle θ relative to an axis of the aimer arm 14. The angle θ can generally be between about 0 and 45 degrees and can be chosen such that the field of view of the image capturing element 32 includes the surgical site 45 and the aiming feature 52. In some cases, the angle θ can be varied before or during surgery to provide the surgeon with an optimal viewing angle of the surgical site 45.

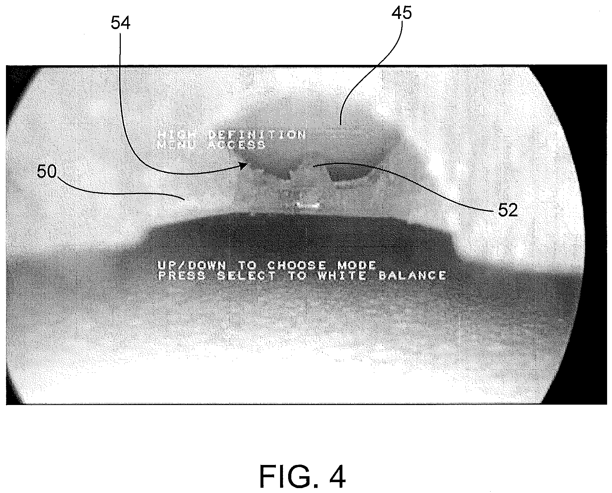

FIG. 4 shows a sample image captured by the image capturing element 32 during positioning of the target 50 and the aiming feature 52 at the surgical site 45. The visible portions of the sample image indicate the field of view of the image capturing element 32. Through the viewing window 54 of the target 50, the aiming feature 52 and the bone surface of the surgical site 45 can be seen. Prior to inserting the surgical instrument 16, the surgeon can thus position the aiming feature 52 on the bone surface to determine the trajectory of the bone passage that will be created by the surgical instrument 16.

During acetabular drilling of, for example, the acetabulum 42, the surgeon should take care not to puncture or damage the cartilage 40. To limit damage to the cartilage 40 with the drill 36, the guide assembly 10 includes one or more depth limiting elements, or drill stops. For example, the target 50 includes a drill stop region 56 that surrounds the aiming feature 52 (FIG. 3A). In use, the user drills through the subchondral bone with the drill 36, and the drill 36 makes contact with the target 50 after exiting through the bone surface. At this point, further distal movement of the drill 36 past the target 50 is prevented or limited when the drill stop region 56 makes contact with the tip of the drill 36. Alternatively, or additionally, another depth limiting element can be coupled to the guide 12, as discussed further below.

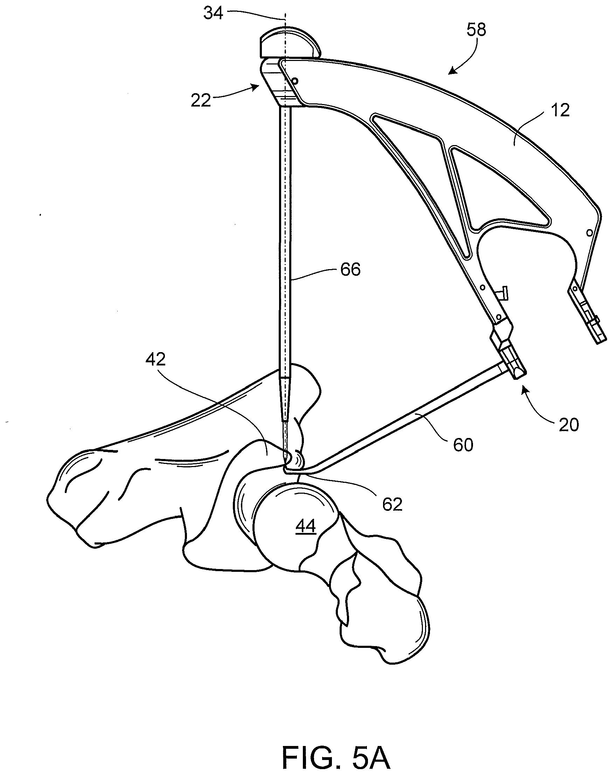

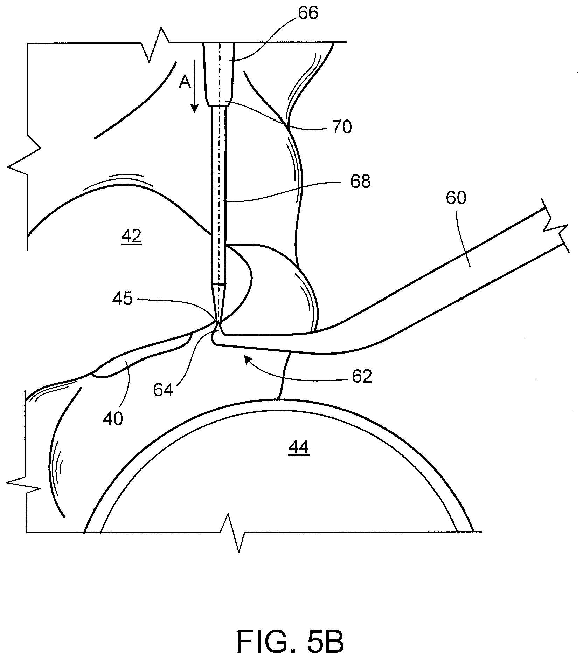

Referring to FIGS.5A and 5B, in an alternative implementation, a guide assembly 58 includes an aimer arm 60 that is coupled to the first attachment portion 20. The aimer arm 60 includes a bent distal portion 62 with an aiming feature 64 that is configured or adapted to contact the bone surface of the acetabulum 42. The aiming feature 64 can be a spike that can engage the bone surface to provide additional stability during the acetabular drilling procedure, as discussed below. A movable bullet 66 is coupled to the second attachment portion 22, and a drill 68 is movably disposed within a cannula of the bullet 66. The guide 12 ensures that the longitudinal axis 34 defined by the bullet 66 and the drill 68 is substantially co-radial with the aiming feature 64. The guide assembly 58 having the aimer arm 60 and the drill 68 can be used, as described above with respect to the guide assembly 10, to drill through the subchondral bone of the acetabular cup 42 from outside the joint to an area inside the joint during the acetabular drilling procedure.

In use, the surgeon inserts the aimer arm 60 into the joint area between the acetabulum 42 and the femoral head 44. Conventional techniques, for example, fluoroscopy or direct arthroscopic visualization, can be used to help position the aiming feature 64 of the aimer arm 60 at the desired surgical site 45. In some cases, the aiming feature 64 is positioned at the bone surface of the acetabulum 42 where a portion of the cartilage 40 is missing or has been surgically removed. After identifying the surgical site 45 at which to drill the bone passage, the surgeon can engage the surgical site 45 with the aiming feature 64 such that unwanted movement of the aimer arm 60 relative to the bone surface of the acetabulum 42 is minimized.

Following the placement of the aiming feature 64 at the desired surgical site 45, the drill 68 can be used to drill through the subchondral bone of the acetabulum 42 from outside the joint to an area inside the joint (FIG. 5B). During the drilling process, the bullet 66 can provide enhanced stability by minimizing a movement of the guide assembly 58 relative to the surgical site 45. For example, as indicated by arrow A in FIG. 5B, the movable bullet 66 can be pushed distally such that a distal tip 70 of the bullet 66 contacts an upper bone surface of the acetabular cup 42. Together with the aiming feature 64, which is engaged at the bottom surface of the acetabular cup 42, the bullet 66 and the aimer arm 60 can generate a clamping force directed towards the surgical site 45. In turn, this clamping force can help further stabilize the guide assembly 58 during acetabular drilling by minimizing unwanted movement. Additionally, the bullet 66 can be configured with a locking or ratcheting mechanism such that the bullet 66 can only move distally to provide this clamping force. Alternatively, or additionally, a clamping force can be generated between a tip of the drill 68 and the aiming feature 64 as the drill 68 moves toward the aiming feature 64 while drilling.

Referring again to FIGS. 3A and 3B, a clamping force can be generated between the bullet 38 and the target 50 as described above with respect to the guide assembly 58. Additionally, or alternatively, the bullet 38 can have a sharp tip 72 that can dig into the upper surface of the acetabulum 42 to provide enhanced stability during drilling.

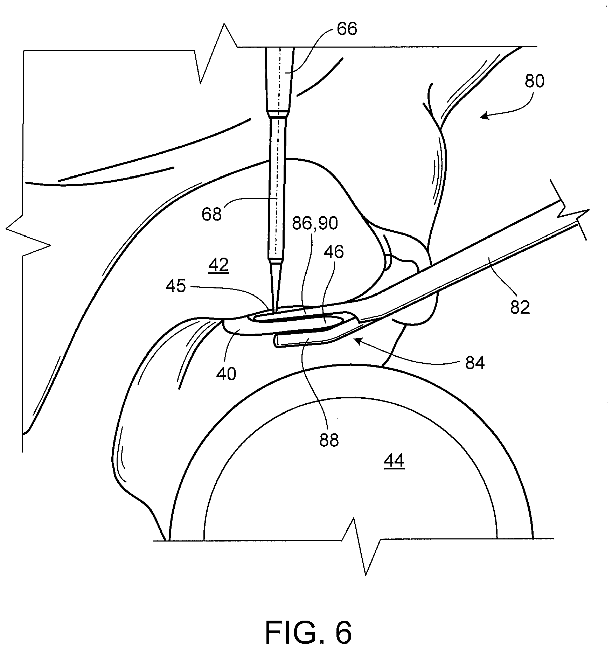

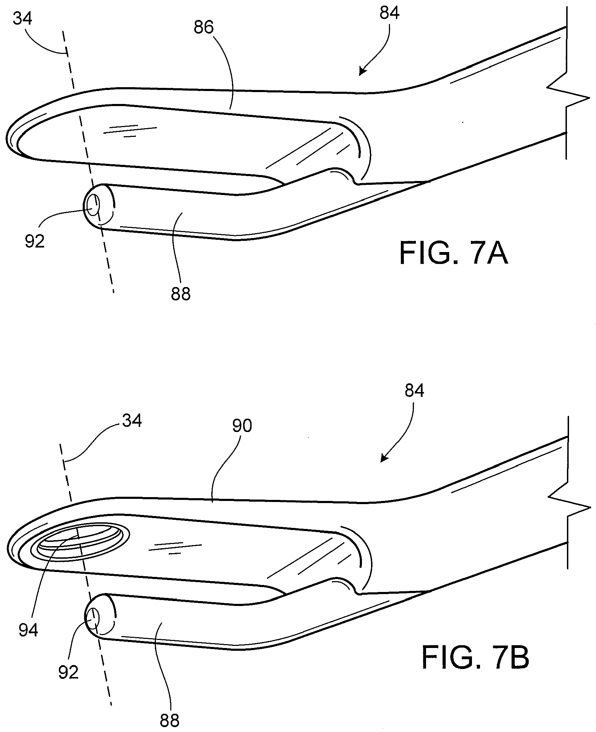

Referring to FIGS. 6 and 7, in an alternative implementation, a guide assembly 80 includes an aiming arm 82 having a distal portion 84 that is shaped to surround the delaminated cartilage portion 46 during drilling surgery of the acetabulum 42. The distal portion 84 includes an aiming feature 86, 90 and an aimer extension 88. The aimer extension 88 extends from the distal portion 84 and is offset from the aiming feature 86, 90 in a distal direction along the longitudinal axis 34.

In use, the surgeon inserts the aimer arm 82 in the joint area between the acetabulum 42 and the femoral head 44 and positions the distal portion 84 such that the aiming feature 86 contacts the bone surface of the acetabulum 42 at the surgical site 45. The delaminated cartilage portion 46 is positioned between the aiming feature 86 and the aimer extension 88 to protect the delaminated cartilage portion 46 during subsequent drilling of the acetabulum 42. As described above with respect to the guide assemblies 10 and 58, the bullet 66 and the aiming feature 86 can generate a clamping force therebetween to further stabilize the guide assembly 80 relative to the acetabulum 42. In this implementation, the aiming feature 86 can also serve as a depth stop for the drill 68 since the aiming feature 86 blocks the drill 68 from moving distally past the aiming feature 86 where the drill 68 could possibly damage the delaminated cartilage portion 46. The aiming feature 86 can further include various aiming indicia such as a laser mark or a spike. Alternatively, or additionally, the aiming feature 86 can include an aperture 94 (FIG. 7B).

In addition to providing, for example, a resting surface for the delaminated cartilage portion 46 during the acetabular drilling procedure, the aimer extension 88 can provide the surgeon with additional means for precisely placing the drilling trajectory of the drill 68 at the surgical site 45. As shown in FIGS. 7A and 7B, the aimer extension 88 is a thin, rod-like structure having a tip 92 that terminates at an intersection point with the longitudinal axis 34 defined by the drill 68. During surgery, because the aiming feature 86 is slipped into a narrow region between the acetabulum 42 and the delaminated cartilage 46, the surgeon may have difficulty visualizing, for example via fluoroscopy or direct arthroscopic visualization, the position of the aimer feature 86 relative to the desired surgical site 45. The aimer extension 88, which is positioned below the cartilage 40, thus serves as an additional indicator for the surgeon to rely on for visualizing the trajectory of the bone passage created by the drill 68. Although the tip 92 of the aimer extension 88 intersects the longitudinal axis 34, the tip 92 will not come in contact with the drill 68 because the drill 68 is blocked from travelling past the aimer feature 86.

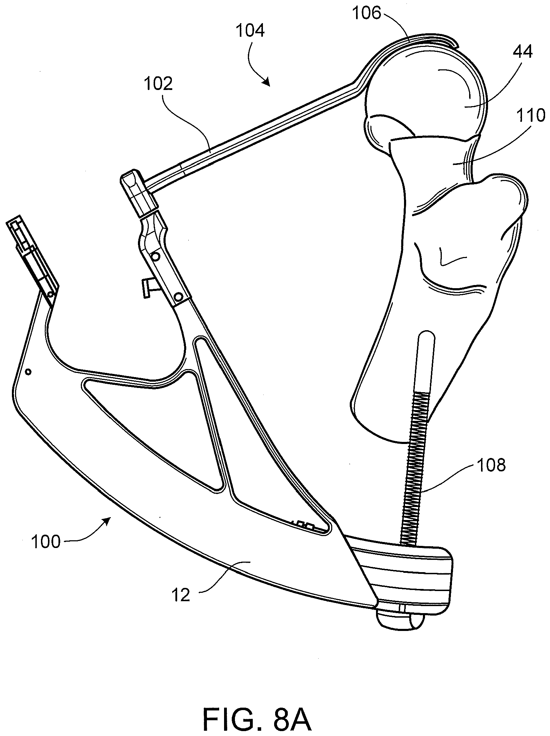

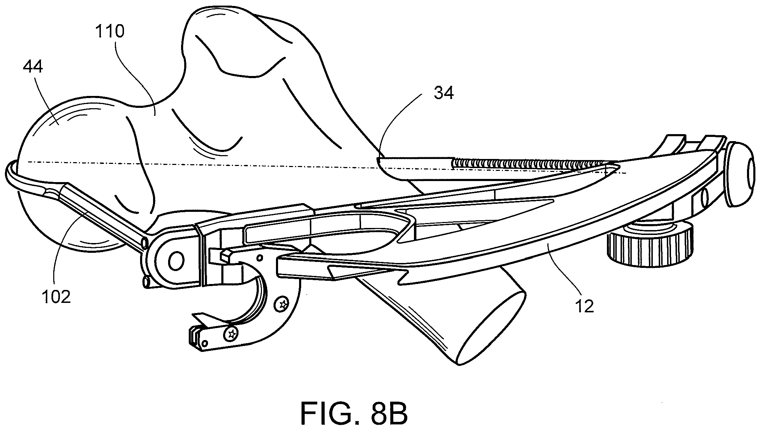

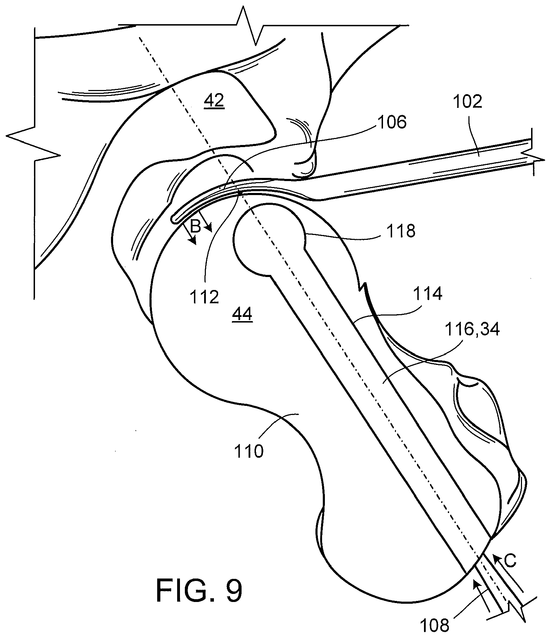

Referring to FIGS. 8A and 8B, in an alternative implementation, a guide assembly 100 can be used to treat femoral head avascular necrosis (AVN) by, for example, drilling through a femoral neck 110 toward the femoral head 44 and removing necrossed bone underneath the joint cartilage. The guide assembly 100 includes an aimer arm 102 with a distal portion 104, the distal portion 104 having a contoured target portion 106. The contoured target portion 106 includes an aiming feature 112 (FIG. 9), for example a laser mark, a spike, or an aperture, and is shaped to substantially conform to a contour of the femoral head 44. The longitudinal axis 34 is substantially co-radial with the aiming feature 112 as described further below.

Referring also to FIG. 9, in use, the aimer arm 102 is placed into the joint area between the acetabulum 42 and the femoral head 44 such that the contoured target portion 106 contacts a surface of the femoral head 44 near the AVN site. The surgeon positions the target portion 106 over the AVN site by inserting the aimer arm 102 and feels for a softening of the subchondral bone of the femoral head 44, indicative of deflection of cartilage overlying the AVN site. Alternatively, or additionally, fluoroscopy, direct arthroscopic visualization, or the like can be used to position the target portion 106 over the AVN site. When using such visualization methods, the aiming feature 112 can be precisely located with respect to the AVN site in a plane of visualization. Positioning in and out of the visualization plane can be accomplished by placing the target portion 106 over the femoral head 44 as described above.

The contoured shape of the target portion 106 of the aimer arm 102 helps minimize unwanted lateral movement of the target portion 106 relative to the femoral head 44 both before and during the drilling process discussed below. For further stabilizing the guide assembly 100 relative to the femoral head 44, and as described above with respect to the guide assemblies 10, 58, and 80, a clamping force can be generated between the target portion 106 and a bullet 108. To help prevent the aimer arm 102 from slipping off the femoral head 44 under increasing clamping force between the target portion 106 and the bullet 108, a distal portion of the target portion 106 extends beyond the longitudinal axis 34 while continuing to conform to the surface of the femoral head 44. As a result, the clamping force between the target portion 106 and the bullet 108 is generated substantially between point C at a distal tip of the bullet 108 and point B on the target portion 106 distal of the aiming feature 112 with which the longitudinal axis 34 is co-radial. Thus, increasing clamping force between points B and C does not translate into lateral forces that can cause the aimer arm 102 to slip off the femoral head 44.

Following positioning of the target portion 106 and stabilizing of the guide assembly 100 as discussed above, the surgeon can remove necrossed bone from the AVN site by drilling a bone hole 114 through the femoral neck 110 and a portion of the femoral head 44. The bone hole 114 defines a drilling axis 116 that is co-linear with the longitudinal axis 34 and can include a larger diameter AVN area 118 at its distal portion. One or more types of drills of varying diameters, including an acorn drill, can be used to create the bone hole 114 and the AVN area 118. Once the necrossed bone is removed, viable bone or bone graft substitute may be packed into the AVN area 118 and/or the bone hole 114.

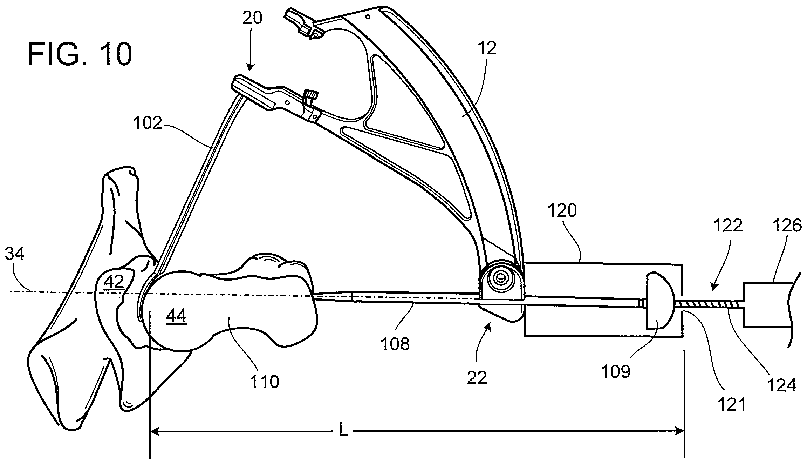

For the AVN implementation discussed above, a depth limiting element located at the aimer arm 102, similar to the drill stop region 56 (FIG. 3A), may not be desirable since it is preferred that the bone hole 114 not extend completely through the surface of the femoral head 44. Thus, alternative to or in addition to depth limiting elements located at the aimer arm, a depth limiting element, such as a depth stop frame 120 (FIG. 10), can be coupled to the guide 12.

Referring to FIG. 10, the depth stop frame 120 is coupled to the second attachment portion 22 at a proximal side of the guide 12. Together with the aimer arm 102 and the guide 12, the depth stop frame 120 defines a fixed length L between a distal portion of the depth stop frame 120 and the target portion 106 (FIG. 9) of the aimer arm 102. The depth stop frame 120 can be any structural element that allows the bullet 108, or the like, to move freely while restricting a distal movement of, for example, a drill 122 disposed within the bullet 108. For example, the depth stop frame 120 can include a tubular structure that surrounds a handle portion 109 of the bullet 108. A reduced diameter portion 121 at a distal portion of the depth stop frame 120 can be sized to allow a thin portion 124 of a drill 122 to pass through while blocking passage of a thick portion 126 of the drill 122. Thus, if a total length of the thin portion 124 of the drill 122 is less than the fixed length L defined by the depth stop 120, the drill 122 cannot reach the target portion 106 (FIG. 9) and will not be able to drill through the surface of the femoral head 44.

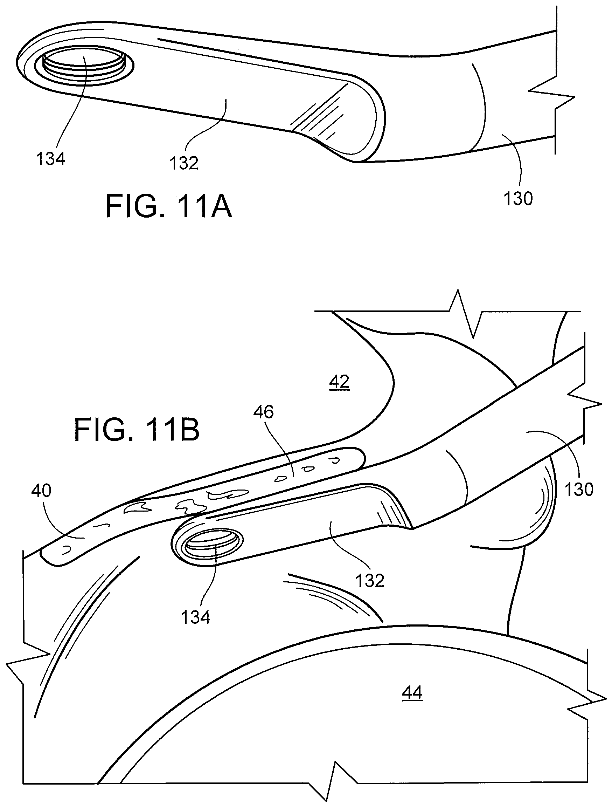

Referring to FIGS. 11A and 11B, in an alternative implementation, an aimer arm 130 includes an aiming feature 132 at its distal end. The aiming feature 132 further includes an aiming aperture 134, which can be an opening or an optically transparent region in the aiming feature 132. The aimer arm 130 can be used during, for example, drilling surgery of the acetabulum 42 as described above and can be coupled to the first attachment portion 20 of the guide 12 (FIG. 1) such that the longitudinal axis 34 of the surgical instrument 16 (FIG. 1) is substantially co-radial with the aiming aperture 134. In use, the aimer arm 130 is configured to be placed at a femoral head-facing surface of the delaminated cartilage portion 46, with no other structure being placed in between the delaminated cartilage portion 46 and the surface of the acetabulum 42. To prevent damage to the cartilage portion 40, during surgery, the surgeon can arthroscopically view and monitor the surface of the cartilage portion 40 through the aiming aperture 134 as the bone passage is drilled. A movement of the cartilage surface indicates to the surgeon that the drill is about to break through the cartilage 40. Stopping the drilling process immediately upon detection of movement in the cartilage surface would thus ensure that the drill does not completely puncture the cartilage 40.

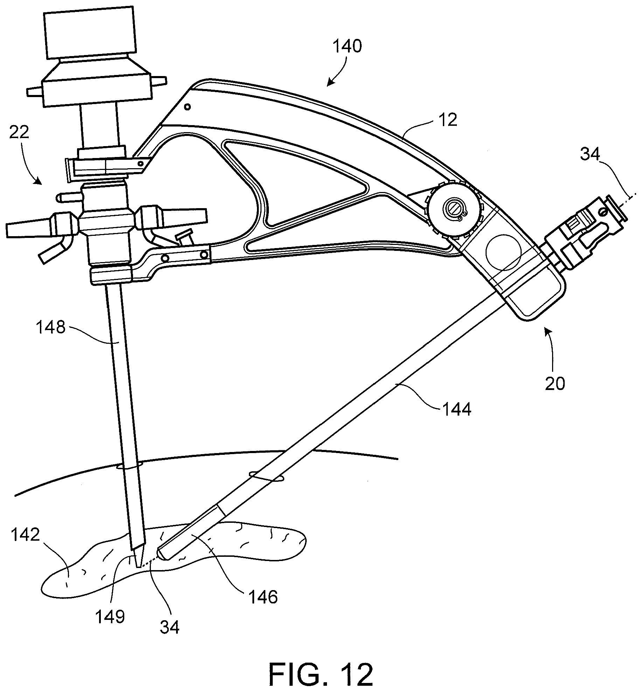

Referring to FIG. 12, a guide assembly 140 includes the guide 12, a motorized shaver 144 attached to the second attachment portion 20, and an endoscope 148 attached to the first attachment portion 22. The motorized shaver 144 includes a shaver blade tip 146 that can be used to, for example, clear out bursa in the greater trochanteric space. Because the bursa is generally full of inflamed tissue, it can be difficult for the surgeon to visualize even a short distance arthroscopically. By rigidly connecting, via the guide 12, the shaver 144 to the endoscope 148 such that the shaver tip 146 is directly in front of a lens end 149 of the endoscope 148, the surgeon can arthroscopically view the immediate area in front of the lens end 149 where tissue is being removed by the shaver tip 146. In some cases, the longitudinal axis 34 defined by the shaver 144 is co-radial with the lens end 149. While it may be desirable in certain surgical applications to position the lens end 149 with an offset, for example 1 cm, relative to the longitudinal axis 34 for improved focusing, such offset may or may not be used for bursa removal applications using the guide assembly 140. In some cases, for example when the shaver tip 146 is a side-cutting, as opposed to end-cutting, blade, the shaver tip 146 may come in contact with the lens end 149 of the endoscope 148. Additionally, the motorized shaver 144 may be movably and lockably disposed within a bore (not shown) that attaches to the second attachment portion 20. For example, the surgeon may first create viewing space around the lens end 149 by operating the guide assembly 140 with the shaver tip 146 locked in a location directly in front of the lens end 149. Once the surgeon can better view the operating area near the lens end 149, the surgeon can then unlock the motorized shaver 144 such that it can piston in and out along and rotate about the longitudinal axis 34 to quickly remove more tissue materials.

While this document contains many specific implementation details, these should not be construed as limitations on the scope of any implementations or of what may be claimed, but rather as descriptions of features specific to particular implementations. For example, while a portion of the aimer arm has been described as extending beyond the longitudinal axis of the surgical instrument, in other implementations, a portion of the aimer arm can extend to a point short of the longitudinal axis, proximate the longitudinal axis, or to a point substantially aligned with the longitudinal axis. Certain features that are described in this document in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination. Thus, while particular implementations of the subject matter have been described, other implementations are within the scope of the following claims.

## Claims

1. A guide assembly (100) comprising:
a guide having a first attachment portion and a second attachment portion;
an aimer arm (102) coupled to the first attachment portion of the guide and having a distal portion (104), the distal portion comprising:
a target portion (106) having an upper surface and a lower surface, and
an aiming feature (112) positioned on the target portion (106) and configured to contact a bone surface;
an image capturing element coupled to the aimer arm and having a viewing direction toward the lower surface of the target portion, such that a field of view of the image capturing element includes a portion of the bone surface and a portion of the aiming feature;
a surgical instrument coupled to the second attachment portion of the guide and configured to drill through bone, the surgical instrument defining a longitudinal axis (34) that is substantially co-radial with the aiming feature;
one or more depth limiting elements configured to limit movement of a distal end of the surgical instrument beyond a preset location along the longitudinal axis; and **characterised in that**
the target portion (106) defines a viewing window (54) through which the image capturing element obtains a view of the portion of the bone surface and the portion of the aiming feature.

2. The guide assembly of claim 1, wherein the aiming feature comprises one or more of a spike, a laser mark, or an aperture.

3. The guide assembly of claims 1 or 2, wherein the one or more depth limiting elements comprise an area about the aiming feature, the area configured to receive a distal end of the surgical instrument

4. The guide assembly of claims 1 or 2, wherein the one or more depth limiting elements comprise a depth stop frame (120) coupled to the guide, the depth stop frame configured to prevent a wide portion of the surgical instrument from advancing past the depth stop frame (120).

5. The guide assembly of any one of claims 1 to 4, wherein the surgical instrument further comprises an outer sleeve (38,66,108), the outer sleeve coupled to the guide and configured to contact a second bone surface, the second bone surface being generally opposite the bone surface that is contacted by the aiming feature of the aimer arm.

6. The guide assembly of any one of claims 1 to 5, wherein the distal portion of the aimer arm further comprises an aimer extension (88), the aimer extension extending from the distal portion (30,104) of the aimer and being offset from the aiming feature such that a distal tip of the aimer extension lies along the longitudinal axis of the surgical instrument.

7. The guide assembly of any one of claims 1 to 6, wherein the distal portion of the aimer arm is contoured to substantially conform to a curved bone surface.

## Patentansprüche

1. Eine Führungsanordnung (100), die Folgendes beinhaltet:
eine Führung mit einem ersten Anbringungsabschnitt und einem zweiten Anbringungsabschnitt;
einen Richterarm (102), der mit dem ersten Anbringungsabschnitt der Führung gekoppelt ist und einen distalen Abschnitt (104) aufweist, wobei der distale Abschnitt Folgendes beinhaltet:
einen Zielabschnitt (106) mit einer oberen Oberfläche und einer unteren Oberfläche und
ein Richtmerkmal (112), das auf dem Zielabschnitt (106) positioniert und konfiguriert ist, um eine Knochenoberfläche zu berühren;
ein Bilderfassungselement, das mit dem Richterarm gekoppelt ist und eine Sichtrichtung in Richtung der unteren Oberfläche des Zielabschnitts aufweist, so dass ein Sichtfeld des Bilderfassungselements einen Abschnitt der Knochenoberfläche und einen Abschnitt des Richtmerkmals umfasst;
ein chirurgisches Instrument, das mit dem zweiten Anbringungsabschnitt der Führung gekoppelt und konfiguriert ist, um durch Knochen zu bohren, wobei das chirurgische Instrument eine Längsachse (34) definiert, die im Wesentlichen co-radial zu dem Richtmerkmal ist;
ein oder mehrere Tiefenbeschränkungselemente, die konfiguriert sind, um die Bewegung eines distalen Endes des chirurgischen Instruments über eine vorgegebene Stelle entlang der Längsachse hinaus zu beschränken; und
**dadurch gekennzeichnet, dass**
der Zielabschnitt (106) ein Sichtfenster (54) definiert, durch welches das Bilderfassungselement eine Sicht auf den Abschnitt der Knochenoberfläche und den Abschnitt des Richtmerkmals erhält.

2. Führungsanordnung gemäß Anspruch 1, wobei das Richtmerkmal eines oder mehrere von einem Dorn, einer Lasermarkierung oder einer Öffnung beinhaltet.

3. Führungsanordnung gemäß Anspruch 1 oder 2, wobei das eine oder die mehreren Tiefenbeschränkungselemente einen Bereich um das Richtmerkmal herum beinhalten, wobei der Bereich konfiguriert ist, um ein distales Ende des chirurgischen Instruments aufzunehmen.

4. Führungsanordnung gemäß Anspruch 1 oder 2, wobei das eine oder die mehreren Tiefenbeschränkungselemente einen mit der Führung gekoppelten Tiefenanschlagrahmen (120) beinhalten, wobei der Tiefenanschlagrahmen konfiguriert ist, um einen breiten Abschnitt des chirurgischen Instruments davon abzuhalten, sich an dem Tiefenanschlagrahmen (120) vorbei vorwärts zu bewegen.

5. Führungsanordnung gemäß einem der Ansprüche 1 bis 4, wobei das chirurgische Instrument ferner eine äußere Hülle (38, 66, 108) beinhaltet, wobei die äußere Hülle mit der Führung gekoppelt und konfiguriert ist, um eine zweite Knochenoberfläche zu berühren, wobei die zweite Knochenoberfläche der von dem Richtmerkmal des Richterarms berührten Knochenoberfläche im Allgemeinen entgegengesetzt ist.

6. Führungsanordnung gemäß einem der Ansprüche 1 bis 5, wobei der distale Abschnitt des Richterarms ferner eine Richterverlängerung (88) beinhaltet, wobei sich die Richterverlängerung von dem distalen Abschnitt (30, 104) des Richters erstreckt und von dem Richtmerkmal versetzt ist, so dass eine distale Spitze der Richterverlängerung entlang der Längsachse des chirurgischen Elements liegt.

7. Führungsanordnung gemäß einem der Ansprüche 1 bis 6, wobei der distale Abschnitt des Richterarms konturangepasst ist, um im Wesentlichen mit einer gekrümmten Knochenoberfläche übereinzustimmen.

## Revendications

1. Un assemblage formant guide (100) comprenant :
un guide ayant une première portion d'attache et une deuxième portion d'attache ;
un bras de dispositif de visée (102) couplé à la première portion d'attache du guide et ayant une portion distale (104), la portion distale comprenant :
une portion cible (106) ayant une surface supérieure et une surface inférieure, et une caractéristique de visée (112) positionnée sur la portion cible (106) et configurée pour être en contact avec une surface osseuse ;
un élément de capture d'images couplé au bras de dispositif de visée et ayant une direction de visualisation vers la surface inférieure de la portion cible, de telle sorte qu'un champ de visualisation de l'élément de capture d'images inclue une portion de la surface osseuse et une portion de la caractéristique de visée ;
un instrument chirurgical couplé à la deuxième portion d'attache du guide et configuré pour percer à travers de l'os, l'instrument chirurgical définissant un axe longitudinal (34) qui est substantiellement coradial avec la caractéristique de visée ;
un ou plusieurs éléments de limite de profondeur configurés pour limiter le déplacement d'une extrémité distale de l'instrument chirurgical au-delà d'un emplacement préétabli le long de l'axe longitudinal ; et
**caractérisé en ce que**
la portion cible (106) définit une fenêtre de visualisation (54) à travers laquelle l'élément de capture d'images obtient une visualisation de la portion de la surface osseuse et de la portion de la caractéristique de visée.

2. L'assemblage formant guide de la revendication 1, dans lequel la caractéristique de visée comprend un ou plusieurs éléments parmi une pointe, une marque laser, ou une ouverture.

3. L'assemblage formant guide des revendications 1 ou 2, dans lequel ces un ou plusieurs éléments de limite de profondeur comprennent une zone autour de la caractéristique de visée, la zone étant configurée pour recevoir une extrémité distale de l'instrument chirurgical.

4. L'assemblage formant guide des revendications 1 ou 2, dans lequel ces un ou plusieurs éléments de limite de profondeur comprennent un cadre de butée de profondeur (120) couplé au guide, le cadre de butée de profondeur étant configuré pour empêcher une portion large de l'instrument chirurgical d'avancer en dépassant le cadre de butée de profondeur (120).

5. L'assemblage formant guide de n'importe laquelle des revendications 1 à 4, dans lequel l'instrument chirurgical comprend en outre un manchon externe (38, 66, 108), le manchon externe étant couplé au guide et configuré pour être en contact avec une deuxième surface osseuse, la deuxième surface osseuse étant généralement opposée à la surface osseuse qui est mise en contact par la caractéristique de visée du bras de dispositif de visée.

6. L'assemblage formant guide de n'importe laquelle des revendications 1 à 5, dans lequel la portion distale du bras de dispositif de visée comprend en outre une extension de dispositif de visée (88), l'extension de dispositif de visée s'étendant depuis la portion distale (30, 104) du dispositif de visée et étant décalée de la caractéristique de visée de telle sorte qu'un bout distal de l'extension de dispositif de visée repose le long de l'axe longitudinal de l'instrument chirurgical.

7. L'assemblage formant guide de n'importe laquelle des revendications 1 à 6, dans lequel la portion distale du bras de dispositif de visée est profilée pour épouser substantiellement une surface osseuse incurvée.
